# FASCICULE DE BREVET EUROPEEN

(11) **EP 2 781 583 B1**
(45) Date de publication et mention de la délivrance du brevet: **08.05.2019**
(21) Numéro de dépôt: 14305339.5
(22) Date de dépôt: 10.03.2014
(51) Int. Cl.: C10G 65/04, C10G 45/64, B01J 29/74, C07C 5/27, C07C 9/22, B01J 21/12, B01J 35/00, B01J 37/00, B01J 35/10

(54) **Procédé de conversion de charges issues de sources renouvelables mettant en oeuvre un catalyseur comprenant une zeolithe nu-10 et une silice alumine**
Verfahren zur Umwandlung von Ladungen aus erneuerbaren Quellen, bei dem ein Katalysator benutzt wird, der einen Zeolith Nu-10 und ein Siliciumdioxid/Aluminiumoxid umfasst
Method for converting loads from renewable sources using a catalyst including an Nu-10 zeolite and a silica-alumina

(30) Priorité: 21.03.2013 FR 1352527
(43) Date de publication de la demande: 24.09.2014
(73) Titulaire: IFP Energies nouvelles, 92500 Rueil-Malmaison (FR)
(72) Inventeur: Marques Mota, Filipe Manuel, 69003 Lyon (FR); Bouchy, Christophe, 69007 Lyon (FR); Duchene, Pascal, 38200 Vienne (FR); Martens, Johan, 3040 Huldenberg (BE)
(74) Mandataire: IFP Energies nouvelles

(56) Documents cités:
- EP-A1- 0 863 198
- EP-A1- 2 138 553
- FR-A1- 2 738 243
- FR-A1- 2 778 582
- FR-A1- 2 926 028

## Description

### Domaine de l'invention

La recherche de nouvelles sources d'énergies renouvelables pour la production de carburants constitue un enjeu majeur. La demande en bases distillats moyens, c'est-à-dire en coupes incorporables au pool kérosène et gazole, est en forte croissance, particulièrement en Europe. L'utilisation de ces nouvelles ressources est un moyen de répondre à cette forte demande, prenant en compte par ailleurs les préoccupations liées à l'environnement

Au sein des différentes voies "alternatives", les bases distillats moyens produites à partir d'une charge paraffinique obtenue à partir d'une charge issue de sources renouvelables, et en particulier d'huiles végétales ou des graisses animales, brutes ou ayant subi un traitement préalable, ainsi que les mélanges de telles charges, présentent des propriétés particulièrement intéressantes. En effet, lesdites charges issues de sources renouvelables contiennent des structures chimiques de type triglycérides ou esters ou acides gras libres, la structure et la longueur de chaîne hydrocarbonée de ces derniers étant compatibles avec les hydrocarbures présents dans les distillats moyens. Lesdites charges issues de sources renouvelables produisent, après hydrotraitement, des charges paraffiniques, exemptes de composés soufrés et de composés aromatiques.

La demande de brevet n° EP 1,681,337 A décrit la transformation de telles charges par décarboxylation, pour former des paraffines ayant un atome de carbone de moins par rapport aux structures chimiques de départ. L'avantage de cette voie tel que décrit dans ce brevet consiste à limiter la consommation d'hydrogène nécessaire. En revanche, les rendements en bases gazole s'en trouvent réduits. Les catalyseurs utilisés sont des catalyseurs métalliques.

Les brevets US 4,992,605 et US 5,705,722 décrivent des procédés de production de bases pour le pool gazole produites à partir de la transformation directe d'huiles végétales (colza, palme, soja, tournesol) ou de biomasse lignocellulosique en hydrocarbures saturés après hydrotraitement ou hydroraffinage de ces produits seuls.

L'effluent liquide issu de ces procédés d'hydrotraitement est essentiellement constitué de n-paraffines qui peuvent présenter des propriétés de tenue à froid insuffisantes pour être incorporées dans un pool gazole et/ou kérosène. De manière à améliorer les propriétés à froid de cet effluent liquide hydrotraité, une étape d'hydroisomérisation est nécessaire pour transformer les n-paraffines en paraffines branchées présentant de meilleures propriétés à froid. De plus, pour un nombre d'atomes de carbone donné, les propriétés à froid d'une paraffine tendent généralement à s'améliorer avec le degré d'isomérisation de ladite paraffine. A titre d'exemple, pour des paraffines à 14 atomes de carbone, la température de fusion du n-tétradécane est de 6°C, les températures de fusion pour le 2-méthyl tridécane et le 3-méthyl tridécane sont respectivement de -26°C et -37°C et la température de fusion pour le 2,3-diméthyl dodécane est de -51 °C. Il est donc également souhaitable de former des isomères multibranchés lors de l'hydroisomérisation. Cette étape d'hydroisomérisation est effectuée sur un catalyseur bifonctionnel présentant à la fois une fonction hydro/déshydrogénante et une fonction acide de Bronsted. Selon le taux d'incorporation et les propriétés à froid visées dans le carburant final, il peut être nécessaire d'effectuer une hydroisomérisation très poussée de l'effluent.

Cette étape d'hydroisomérisation s'accompagne généralement de la production de produits de craquage trop légers pour être incorporés dans un pool gazole et/ou kérosène. Il en résulte donc une perte de rendement qu'il est désirable de minimiser.

Les demandes de brevets EP 2 138 553 et EP 2 138 552 décrivent un procédé de traitement d'une charge issue d'une source renouvelable comprenant un hydrotraitement, éventuellement une séparation gaz/liquide, éventuellement suivie d'une élimination des composés azotés, et une hydroisomérisation en présence d'un catalyseur comprenant au moins un métal du groupe VIII et/ou au moins un métal du groupe VIB et au moins un tamis moléculaire zéolithique 10 MR monodimensionnel, de préférence choisi parmi les tamis moléculaires de type structural TON tel que la Nu-10, EUO choisis parmi la EU-1 et la ZSM-50 seules ou en mélange, ou les tamis moléculaires ZSM-48, ZBM-30, IZM-1, COK-7, EU-2 et EU-11. Lesdits procédés permettent d'obtenir des rendements élevés en base gazole.

La demande de brevet FR 2778582 divulgue un procédé d'hydroisomérisation d'un résidu d'hydrocraquage utilisant un catalyseur comprenant une zéolithe Nu-10 et une silice alumine (Pt-C1 Si et Pt-C1BSi) et une diminution des produits de craquage.

La demande de brevet EP0863198 divulgue un procédé d'hydroisomérisation d'un résidu d'hydrocraquage utilisant un catalyseur comprenant une zéolithe Nu-10 et une alumine.

Les travaux de recherche effectués par le demandeur l'ont conduit à découvrir que, de façon surprenante, l'utilisation d'un catalyseur comprenant au moins une zéolithe Nu-10 et au moins une silice alumine dans un procédé d'hydroconversion d'une charge paraffinique produite à partir de ressources renouvelables, permet de limiter la production de produits craqués légers ne pouvant pas être incorporés dans un pool gazole et/ou kérosène tout en favorisant la production d'isomères multibranchés, le degré de ramification de l'effluent obtenu étant caractéristique d'un effluent présentant des propriétés à froid améliorées par rapport à la charge paraffinique de départ.

Un objectif de la présente invention est donc de fournir un procédé de conversion d'une charge paraffinique constituée par des hydrocarbures ayant un nombre d'atomes de carbone compris entre 9 et 25 et issue de sources renouvelables utilisant un catalyseur comprenant au moins une zéolithe Nu-10 et au moins une silice alumine, ledit catalyseur étant très sélectif en hydroisomérisation desdites paraffines et permettant à la fois de limiter la production de produits craqués légers et de favoriser la production d'isomères multibranchés.

### Objet de l'invention

La présente invention concerne un procédé en continu de conversion d'une charge paraffinique produite à partir de ressources renouvelables en bases distillats moyens, gazole et/ou kerosène.

En particulier, un objet de la présente invention est un procédé de conversion d'une charge paraffinique constituée par des hydrocarbures ayant un nombre d'atomes de carbone compris entre 9 et 25, ladite charge paraffinique étant produite à partir de ressources renouvelables, à l'exclusion des charges paraffiniques obtenues par un procédé mettant en jeu une étape de valorisation par la voie Fischer-Tropsch, ledit procédé mettant en oeuvre un catalyseur comprenant au moins un métal hydro-déshydrogénant choisi dans le groupe formé par les métaux du groupe VIB et du groupe VIII de la classification périodique, pris seuls ou en mélange et un support comprenant au moins une zéolithe Nu-10 et au moins une silice alumine, ledit procédé opérant à une température comprise entre 150 et 500°C, à une pression comprise entre 0,1 MPa et 15 MPa, à une vitesse spatiale horaire comprise entre 0,1 et 10 h⁻¹ et en présence d'une quantité totale d'hydrogène mélangée à la charge telle que le ratio hydrogène/charge soit compris entre 70 et 2000 Nm³/m³ de charge.

Un objet de l'invention est de fournir un procédé de conversion d'une charge paraffinique produite à partir de ressources renouvelables permettant de produire des bases distillats moyens, en particulier une base kérosène et/ou une base gazole, tout en limitant la production de produits légers non incorporables auxdites bases.

Un autre objet de l'invention est d'améliorer le degré de ramification par hydroisomérisation de la charge paraffinique mise en oeuvre et produite à partir de ressources renouvelables, le degré de ramification étant ajusté de manière à obtenir, pour les bases distillats moyens, des propriétés, en particulier à froid, compatibles avec les normes en vigueur pour les distillats moyens.

### Résumé de l'invention.

L'invention porte sur un procédé de conversion d'une charge paraffinique constituée par des hydrocarbures ayant un nombre d'atomes de carbone compris entre 9 et 25, ladite charge paraffinique étant produite à partir de ressources renouvelables, à l'exclusion des charges paraffiniques obtenues par un procédé mettant en jeu une étape de valorisation par la voie Fischer-Tropsch, ledit procédé mettant en oeuvre un catalyseur comprenant au moins un métal hydro-déshydrogénant choisi dans le groupe formé par les métaux du groupe VIB et du groupe VIII de la classification périodique, pris seuls ou en mélange et un support comprenant au moins une zéolithe Nu-10 et au moins une silice alumine, ledit procédé opérant à une température comprise entre 150 et 500°C, à une pression comprise entre 0,1 MPa et 15 MPa, à une vitesse spatiale horaire comprise entre 0,1 et 10 h⁻¹ et en présence d'une quantité totale d'hydrogène mélangée à la charge telle que le ratio hydrogène/charge soit compris entre 70 et 2000 Nm³/m³ de charge.

### Description de l'invention

### La charge

Conformément à l'invention, ladite charge paraffinique constituée par des hydrocarbures ayant un nombre d'atomes de carbone compris entre 9 et 25 utilisée dans le procédé selon l'invention est produite à partir de ressources renouvelables.

De préférence, ladite charge paraffinique est constituée par des hydrocarbures ayant un nombre d'atomes de carbone compris entre 10 et 25 et de préférence entre 10 et 22.

La teneur en paraffines dans ladite charge mise en oeuvre dans le procédé selon l'invention est avantageusement supérieure à 90% poids, de préférence supérieure à 95% poids, de manière encore plus préférée supérieure à 98% poids.

De préférence, ladite charge paraffinique est produite à partir de ressources renouvelables choisies parmi les huiles végétales, les huiles d'algues ou algales, les huiles de poissons et les graisses d'origine végétale ou animale, ou des mélanges de telles charges.

Selon l'invention, ladite charge utilisée dans le procédé selon l'invention est une charge paraffinique produite à partir de ressources renouvelables, à l'exclusion des charges paraffiniques obtenues par un procédé mettant en jeu une étape de valorisation par la voie Fischer-Tropsch. Ainsi, les charges paraffiniques obtenues, selon un procédé de synthèse Fischer-Tropsch, à partir d'un gaz de synthèse (CO+H₂) produit à partir de ressources renouvelables selon la voie BTL aussi appelée selon la terminologie anglo-saxonne "Biomass To Liquid", sont exclues des charges utilisées dans le procédé selon l'invention.

Lesdites huiles végétales peuvent avantageusement être brutes ou raffinées, totalement ou en partie, et issues des végétaux choisis parmi le colza, le tournesol, le soja, le palmier, l'olive, la noix de coco, le coprah, le ricin, le coton, les huiles d'arachides, de lin et de crambe et toutes les huiles issues par exemple du tournesol ou du colza par modification génétique ou hybridation, cette liste n'étant pas limitative. Lesdites graisses animales sont avantageusement choisies parmi le lard et les graisses composées de résidus de l'industrie alimentaire ou issus des industries de la restauration. Les huiles de fritures, les huiles animales variées comme les huiles de poisson, le suif, le saindoux peuvent également être utilisées.

Les ressources renouvelables à partir desquelles est produite la charge paraffinique utilisée dans le procédé selon l'invention contiennent essentiellement des structures chimiques de type triglycérides que l'homme du métier connait également sous l'appellation tri ester d'acides gras ainsi que des acides gras libres, dont les chaînes grasses contiennent un nombre d'atomes de carbone compris entre 9 et 25.

La structure et la longueur de chaîne hydrocarbonée de ces derniers est compatible avec les hydrocarbures présents dans le gazole et le kérosène, c'est à dire la coupe distillats moyens. Un tri ester d'acide gras est ainsi composé de trois chaînes d'acides gras. Ces chaînes d'acide gras sous forme de tri ester ou sous forme d'acide gras libres, possèdent un nombre d'insaturations par chaîne, également appelé nombre de doubles liaisons carbone-carbone par chaîne, généralement compris entre 0 et 3 mais qui peut être plus élevé notamment pour les huiles issues d'algues qui présentent généralement un nombre d'insaturations par chaînes de 5 à 6.

Les molécules présentes dans lesdites ressources renouvelables utilisées dans la présente invention présentent donc un nombre d'insaturations, exprimé par molécule de triglycéride, avantageusement compris entre 0 et 18. Dans ces charges, le taux d'insaturation, exprimé en nombre d'insaturations par chaîne grasse hydrocarbonée, est avantageusement compris entre 0 et 6.

Les ressources renouvelables comportent généralement également différentes impuretés et notamment des hétéroatomes tels que l'azote. Les teneurs en azote dans les huiles végétales sont généralement comprises entre 1 ppm et 100 ppm poids environ, selon leur nature. Elles peuvent atteindre jusqu'à 1% poids sur des charges particulières.

Ladite charge paraffinique utilisée dans le procédé selon l'invention est avantageusement produite à partir de ressources renouvelables selon des procédés connus de l'homme du métier. Une voie possible est la transformation catalytique desdites ressources renouvelables en effluent paraffinique désoxygéné en présence d'hydrogène et en particulier, l'hydrotraitement.

De préférence, ladite charge paraffinique est produite par hydrotraitement desdites ressources renouvelables. Ces procédés d'hydrotraitement de ressources renouvelables sont déjà bien connus et sont décrits dans de nombreux brevets. A titre d'exemple, ladite charge paraffinique utilisée dans le procédé selon l'invention peut avantageusement être produite, de préférence par hydrotraitement puis par séparation gaz/liquide, à partir desdites ressources renouvelables comme dans le brevet FR 2 910 483 ou dans le brevet FR 2 950 895.

De manière préférée, ladite charge paraffinique est produite par hydrotraitement desdites ressources renouvelables, en présence d'un catalyseur en lit fixe, ledit catalyseur comprenant une fonction hydro-déshydrogénante et un support amorphe, à une température comprise entre 200 et 450°C, à une pression comprise entre 1 MPa et 10 MPa, à une vitesse spatiale horaire comprise entre 0,1 h⁻¹ et 10 h⁻¹ et en présence d'une quantité totale d'hydrogène mélangée à la charge telle que le ratio hydrogène/charge soit compris entre 150 et 750 Nm³ d'hydrogène/m³ de charge.

Le catalyseur utilisé dans ladite étape d'hydrotraitement est un catalyseur classique comprenant de préférence, au moins un métal du groupe VIII et/ou du groupe VIB et au moins un support choisi dans le groupe formé par l'alumine, la silice, les silice alumines, la magnésie, les argiles et les mélanges d'au moins deux de ces minéraux. Ce support peut également renfermer d'autres composés et par exemple des oxydes choisis dans le groupe formé par l'oxyde de bore, la zircone, l'oxyde de titane, l'anhydride phosphorique.

### Le catalyseur

Le procédé selon l'invention est un procédé de conversion de ladite charge paraffinique produite à partir de ressources renouvelables mettant en oeuvre un catalyseur comprenant au moins un métal hydro-déshydrogénant choisi dans le groupe formé par les métaux du groupe VIB et du groupe VIII de la classification périodique, pris seuls ou en mélange et un support comprenant au moins une zéolithe Nu-10 et au moins une silice alumine. De préférence, ledit procédé est un procédé d'hydroisomérisation.

Le catalyseur utilisé dans le procédé selon l'invention est avantageusement de type bifonctionnel, c'est-à-dire qu'il possède une fonction hydro/déshydrogénante et une fonction hydroisomérisante.

De préférence, les éléments du groupe VIII sont choisis parmi les métaux nobles et non nobles du groupe VIII et de préférence parmi le fer, le cobalt, le nickel, le ruthénium, le rhodium, le palladium, l'osmium, l'iridium ou le platine, pris seuls ou en mélange et de manière préférée parmi le cobalt, le nickel, le platine et le palladium, pris seuls ou en mélange.

Dans le cas où les éléments du groupe VIII sont choisis parmi les métaux nobles du groupe VIII, les éléments du groupe VIII sont avantageusement choisis parmi le platine et le palladium, pris seuls ou en mélange. Dans ce cas, lesdits éléments sont utilisés sous leur forme réduite.

Dans le cas où les éléments du groupe VIII sont choisis parmi les métaux non nobles du groupe VIII, les éléments du groupe VIII sont avantageusement choisis parmi le cobalt et le nickel, pris seuls ou en mélange. De préférence, les éléments du groupe VIB sont choisis parmi le tungstène et le molybdène, pris seuls ou en mélange. Dans le cas où la fonction hydrogénante comprend un élément du groupe VIII et un élément du groupe VIB, les associations de métaux suivants sont préférées: nickel-molybdène, cobalt-molybdène, fer-molybdène, fer-tungstène, nickel-tungstène, cobalt-tungstène, et de manière très préférée: nickel-molybdène, cobalt-molybdène, nickel-tungstène. Il est également possible d'utiliser des associations de trois métaux tel que par exemple nickel-cobalt-molybdène. Lorsqu'une combinaison de métaux du groupe VI et du groupe VIII est utilisée, le catalyseur est alors préférentiellement utilisé sous une forme sulfurée.

Dans le cas où ledit catalyseur comprend au moins un métal noble du groupe VIII, la teneur en métal noble dudit catalyseur est avantageusement comprise entre 0,01 et 5% en poids de manière préférée entre 0,1 et 4% en poids et de manière très préférée entre 0,1 et 2% en poids par rapport à la masse totale dudit catalyseur.

Selon un mode préféré, ledit catalyseur peut également comprendre de l'étain en plus dudit ou desdits métal(aux) noble(s), le teneur en étain étant de préférence comprise entre 0,1 et 0,5% en poids par rapport à la masse totale de catalyseur.

Dans le cas où le catalyseur comprend au moins un métal du groupe VIB en combinaison avec au moins un métal non noble du groupe VIII, la teneur en métal du groupe VIB est avantageusement comprise entre 5 et 40% en poids d'oxyde par rapport à la masse totale dudit catalyseur, de manière préférée entre 10 et 35% en poids d'oxyde et de manière très préférée entre 15 et 30% en poids d'oxyde et la teneur en métal non noble du groupe VIII est avantageusement comprise entre 0,5 et 10% en poids d'oxyde par rapport à la masse totale dudit catalyseur, de manière préférée entre 1 et 8% en poids d'oxyde et de manière très préférée entre 1,5 et 6% en poids d'oxyde.

Conformément à l'invention, ledit catalyseur comprend un support comprenant au moins une zéolithe Nu-10 et au moins une silice alumine. Selon un mode de réalisation préféré, le support du catalyseur utilisé dans le procédé selon l'invention est constitué d'une zéolithe Nu-10 et d'une silice alumine.

La zéolithe Nu-10 est un solide microporeux cristallisé 10 MR monodimensionnel de type structural TON. Le tableau de diffraction de rayons X de la zéolithe Nu-10 ainsi qu'un protocole de synthèse sont décrits dans le brevet EP0077624B1. Ladite zéolithe Nu-10 présente une composition chimique, exprimée en moles, définie par la formule générale suivante : 0,5 à 1,5 R₂O : Y₂O₃ : au moins 20 XO₂ : 0 à 4000 H₂O, dans laquelle R représente un cation de monovalent ou (1/n) d'un cation de valence n, Y représente au moins un élément choisi parmi l'aluminium, le fer, le chrome, le vanadium, le molybdène, l'arsenic, l'antimoine, le manganèse, le gallium ou le bore, X est l'aluminium et/ou le germanium.

De préférence, la zéolithe Nu-10 est sous forme aluminosilicate, c'est à-dire que l'élément Y est constitué par de l'aluminium, et l'élément X constitué par du silicium.

De préférence, le rapport molaire du nombre d'atomes de silicium sur le nombre d'atomes d'aluminium Si/Al est inférieur à 200, de préférence inférieur à 150, de manière très préférée inférieur à 120. Ladite zéolithe Nu-10 entrant dans la composition du support du catalyseur utilisé dans le procédé selon l'invention est avantageusement au moins en partie, de préférence pratiquement totalement, sous forme acide, c'est-à-dire sous forme acide (H⁺).

Pour cela, lorsque le cation R est inorganique, la zéolithe est avantageusement échangée par au moins un traitement par une solution d'au moins un sel d'ammonium de manière à obtenir la forme ammonium de la zéolithe Nu-10 qui une fois calcinée conduit à la forme acide (H⁺) de ladite zéolithe. Cette étape d'échange peut être effectuée à toute étape de la préparation du catalyseur. Lorsque R est une molécule azotée, la forme acide de ladite zéolithe peut être obtenue par calcination; sans mise en oeuvre de l'étape d'échange préalable. Cette étape de calcination peut être effectuée à toute étape de la préparation du catalyseur.

Les silice alumines utilisées en tant que support dudit catalyseur sont des solides non microporeux constitués par la combinaison intime de silice et d'alumine. On peut obtenir des silice alumines dans le domaine complet de composition entre 0 et 100% d'Al₂O₃. Au delà de la composition chimique globale, le degré d'association du silicium et de l'aluminium, ainsi que les propriétés texturales du solide dépendent fortement de la méthode de synthèse. Différentes méthodes de synthèse peuvent être utilisées pour préparer une silice alumine. Les modes de synthèse diffèrent suivant l'état de genèse des réactifs mis en jeu. Les réactifs aluminiques et/ou siliciques peuvent être plus ou moins préformés à savoir que suivant les modes de synthèse le réactif aluminique sera soit une solution de sel métallique qui est un réactif primaire ou un gel qui est un réactif qu'il est possible de qualifier de « préformé ». Un type de silice alumine est synthétisé par imprégnation d'une alumine par un précurseur silicique préformé (gel de silice). Ainsi le coeur de cette silice alumine est très riche en alumine alors que la surface est riche en silice (W. Daniell, U. Schubert, R. Glöckler, A. Meyer, K. Noweck, H. Knözinger, Applied catalysis A: general, 196, 147 (2000)). Un autre type de silice alumine peut être préparé avec une méthode séquencée. Dans ce cas seul le réactif silicique est préformé ("hydrogel de silice") et le réactif aluminique est une solution aqueuse de sel d'aluminium. Plus précisément, la méthode séquencée consiste à préparer le réactif silicique préformé puis de faire précipiter le sel d'aluminium au contact de l'hydrogel de silice fraîchement préparé. Un hydrogel de silice peut être préparé par acidification de silicate de sodium avec un acide minéral (acide sulfurique). Un sel d'aluminium dilué est ensuite ajouté à cet hydrogel (P.K. Sinhamahapatra, D.K. Sharma, R.P. Mehrotra, J. appl. Chem. Biotechnol., 28, 740 (1978)). Un autre type de silice alumine peut être obtenu par la méthode de cogélification pour laquelle les précurseurs métalliques sont ajoutés simultanément. Ainsi les réactifs mis en présence sont tous deux des solutions de sels métalliques. La cogélification consiste en la précipitation en une étape, soit la co-précipitation, d'une solution aqueuse de silicium et d'une solution aqueuse d'aluminium dans un domaine de pH où les deux précurseurs précipitent. Toute silice alumine connue de l'homme de l'art convient pour l'invention.

Selon la présente invention, la silice alumine utilisée en tant que support dudit catalyseur contient une quantité supérieure à 5% poids et inférieure ou égale à 95% poids de silice, de préférence comprise entre 10 et 80% poids, de manière préférée une teneur en silice supérieure à 20% poids et inférieure à 80% poids et de manière encore plus préférée supérieure à 25% poids et inférieure à 75% poids, la teneur en silice est avantageusement comprise entre 10 et 50% poids. Ladite silice alumine préférée présente avantageusement les caractéristiques texturales suivantes :
- un diamètre moyen poreux, mesuré par porosimétrie au mercure, compris entre 20 et 140 Å,
- un volume poreux total, mesuré par porosimétrie au mercure, compris entre 0,1 ml/g et 0,5 ml/g,
- un volume poreux total, mesuré par porosimétrie azote, compris entre 0,1 ml/g et 0,5 ml/g,
- une surface spécifique BET comprise entre 100 et 550 m²/g,
- un volume poreux, mesuré par porosimétrie au mercure, compris dans les pores de diamètre supérieur à 140 Å inférieur à 0,1 ml/g ,
- un volume poreux, mesuré par porosimétrie au mercure, compris dans les pores de diamètre supérieurs à 500 Å inférieur à 0,1 ml/g,
- un diagramme de diffraction X qui contient au moins les raies principales caractéristiques d'au moins une des alumines de transition comprise dans le groupe composé par les alumines alpha, rhô, chi, eta, gamma, kappa, thêta et delta.

De préférence, ladite silice alumine contient :
- une teneur en impuretés cationiques (par exemple Na⁺) inférieure à 0,1% poids, de manière préférée inférieure à 0,05% poids et de manière encore plus préférée inférieure à 0,025% poids. On entend par teneur en impuretés cationiques la teneur totale en alcalins et alcalino-terreux;
- une teneur en impuretés anioniques (par exemple SO₄²⁻, Cl⁻) inférieure à 1% poids, de manière préférée inférieure à 0,5% poids et de manière encore plus préférée inférieure à 0,1% poids.

Selon un mode de réalisation, le catalyseur utilisé dans le procédé selon l'invention peut avantageusement contenir un liant.

Ledit liant peut avantageusement être amorphe ou cristallisé. De préférence, ledit liant est avantageusement choisi dans le groupe formé par l'alumine, la silice, les argiles, l'oxyde de titane, l'oxyde de bore et la zircone, pris seuls ou en mélange. On peut choisir également les aluminates. De préférence, ledit liant du support est l'alumine. De manière préférée, ledit liant du support est une matrice contenant de l'alumine sous toutes ses formes connues de l'homme du métier, telles que par exemple les alumines de type alpha, gamma, éta, delta. Lesdites alumines diffèrent par leur surface spécifique et leur volume poreux. Ledit liant du support se présente de préférence sous la forme de billes, grains ou extrudés.

De préférence, ledit catalyseur comprend de 5 à 98% en poids de liant, de manière très préférée de 10 à 95% en poids et de manière encore préférée de 20 à 95% en poids par rapport à la masse totale dudit catalyseur.

Dans le cas où ledit catalyseur contient un liant, le catalyseur comprend une teneur totale en zéolithe Nu-10 et en silice alumine, avantageusement comprise entre 1,5% et 94,5%, de manière préférée entre 10% et 80%, de manière très préférée entre 20% et 70% en poids par rapport à la masse totale dudit catalyseur et teneur en poids de la zéolithe Nu-10 est inférieure à la teneur en poids de la silice alumine.

Selon un autre mode de réalisation, le catalyseur utilisé dans le procédé selon l'invention ne contient pas de liant. Dans ce cas, ledit catalyseur comprend avantageusement une teneur totale en zéolithe Nu-10 et en silice alumine d'au moins 50%, de manière préférée d'au moins 57%, de manière très préférée d'au moins 64% en poids par rapport à la masse totale dudit catalyseur et la teneur en poids de la zéolithe Nu-10 est inférieure à la teneur en poids de la silice alumine.

Selon un mode de réalisation préférée, le support est constitué d'une silice alumine et d'une zéolithe Nu-10.

Selon un autre mode de réalisation préférée, le support est constitué d'une silice alumine, d'une zéolithe Nu-10 et d'un liant.

Le support comprenant au moins une zéolite Nu-10 et au moins une silice alumine est avantageusement préparé selon toutes les méthodes bien connues de l'homme du métier à partir des solides préparés comme décrit plus haut.

La zéolithe Nu-10 peut avantageusement être introduite selon toute méthode connue de l'homme du métier et ce à tout stade de la préparation du support ou du catalyseur.

Un procédé préféré de préparation du catalyseur selon la présente invention comprend avantageusement les étapes décrites ci dessous.

Selon un mode de préparation préféré, la zéolithe Nu-10 peut avantageusement être introduite au cours de la synthèse des précurseurs de la silice alumine. La zéolithe Nu-10 peut être, sans que cela soit limitatif, par exemple sous forme de poudre, poudre broyée, suspension, suspension ayant subi un traitement de désagglomération. Ainsi, par exemple, la zéolithe Nu-10 peut avantageusement être mise en suspension acidulée ou non à une concentration ajustée à la teneur finale en zéolithe visée sur le support. Cette suspension appelée couramment une barbotine est alors mélangée avec les précurseurs de la silice-alumine à un stade quelconque de sa synthèse comme décrite plus haut. Selon un autre mode de préparation préféré, la zéolithe Nu-10 et la silice alumine peuvent avantageusement être introduites également lors de la mise en forme du support avec éventuellement au moins un liant. La zéolithe Nu-10 et la silice alumine peuvent avantageusement être, sans que cela soit limitatif, sous forme de poudre, poudre broyée, suspension, suspension ayant subi un traitement de désagglomération.

Avantageusement, de la zéolithe Nu-10 et de la silice alumine sous forme de poudres sont mélangées puis le mélange est mis en forme.

La mise en forme peut être réalisée par toute technique connue de l'homme du métier comme par exemple l'extrusion, le pastillage, la mise en forme sous forme de billes au drageoir tournant ou au tambour, la coagulation en goutte, "oil-drop", "oil-up", la dragéification.

Les supports ainsi obtenus sont mis en forme sous la forme de grains de différentes formes et dimensions. Ils sont utilisés en général sous la forme d'extrudés cylindriques ou polylobés tels que bilobés, trilobés, polylobés de forme droite ou torsadée, mais peuvent éventuellement être fabriqués et employés sous la forme de poudres concassées, de tablettes, d'anneaux, de billes, de roues. Après mise en forme, le support du catalyseur utilisé dans le procédé selon la présente invention peut avantageusement être soumis à différents traitements thermiques. Le support peut tout d'abord être soumis à une étape de séchage. Ladite étape de séchage est avantageusement effectuée par toute technique connue de l'homme du métier.

De préférence, le séchage est effectué sous flux d'air. Ledit séchage peut également être avantageusement effectué sous flux de tout gaz oxydant, réducteur ou inerte. De préférence, le séchage est avantageusement effectué entre 50 et 180°C, de manière préférée entre 60 et 150°C et de manière très préférée entre 80 et 130°C.

Ledit support, éventuellement séché, subit ensuite de préférence, une étape de calcination.

Ladite étape de calcination est avantageusement réalisée en présence d'oxygène moléculaire, par exemple en effectuant un balayage d'air, à une température avantageusement supérieure à 200°C et inférieure ou égale à 1100°C. Ladite étape de calcination peut avantageusement être effectuée en lit traversé, en lit léché ou en atmosphère statique. Par exemple, le four utilisé peut être un four rotatif tournant ou être un four vertical à couches traversées radiales. De préférence, ladite étape de calcination est effectuée entre plus d'une heure à 200°C à moins d'une heure à 1100°C. La calcination peut avantageusement être opérée en présence de vapeur d'eau et/ou en présence d'une vapeur acide ou basique. Par exemple, la calcination peut être réalisée sous pression partielle d'ammoniaque.

Des traitements post-calcination peuvent éventuellement être effectués, de manière à améliorer les propriétés du support, par exemple texturales.

Le support comprenant la zéolithe Nu-10, la silice alumine et éventuellement un liant peut ainsi être éventuellement soumis à un traitement hydrothermal en atmosphère confinée. On entend par traitement hydrothermal en atmosphère confinée un traitement par passage à l'autoclave en présence d'eau à une température supérieure à la température ambiante.

Au cours de ce traitement hydrothermal, on peut avantageusement traiter le support. Ainsi, on peut avantageusement imprégner le support, préalablement à son passage à l'autoclave, l'autoclavage étant fait soit en phase vapeur, soit en phase liquide, cette phase vapeur ou liquide de l'autoclave pouvant être acide ou non. Cette imprégnation, préalable à l'autoclavage, peut avantageusement être acide ou non. Cette imprégnation, préalable à l'autoclavage peut avantageusement être effectuée à sec ou par immersion du support dans une solution aqueuse acide. Par imprégnation à sec, on entend mise en contact du support avec un volume de solution inférieur ou égal au volume poreux total du support. De préférence, l'imprégnation est réalisée à sec.

L'autoclave est de préférence un autoclave à panier rotatif tel que celui défini dans la demande brevet EP-A-0 387 109.

La température pendant l'autoclavage peut être comprise entre 100 et 250°C pendant une période de temps comprise entre 30 minutes et 3 heures.

La fonction hydro-déshydrogénante peut avantageusement être introduite à toute étape de la préparation du catalyseur, de manière très préférée après mise en forme du support constitué par la zéolithe Nu-10, la silice alumine et éventuellement un liant. La mise en forme est avantageusement suivie d'une calcination, la fonction hydro-déshydrogénante peut également avantageusement être introduite avant ou après cette calcination. La préparation se termine généralement par une calcination à une température de 250 à 600°C. Une autre des méthodes préférées selon la présente invention consiste avantageusement à mettre en forme le support après un malaxage de ce dernier, puis passage de la pâte ainsi obtenue au travers d'une filière pour former des extrudés. La fonction hydro-déshydrogénante peut avantageusement être alors introduite en partie seulement ou en totalité, au moment du malaxage. Elle peut également avantageusement être introduite par une ou plusieurs opérations d'échange ionique sur le support calciné.

D'une façon préférée, le support est imprégné par une solution aqueuse. L'imprégnation du support est de préférence effectuée par la méthode d'imprégnation dite "à sec" bien connue de l'homme du métier. L'imprégnation peut avantageusement être effectuée en une seule étape par une solution contenant l'ensemble des éléments constitutifs du catalyseur final.

La fonction hydro-déshydrogénante peut avantageusement être introduite par une ou plusieurs opérations d'imprégnation du support mis en forme et calciné, par une solution contenant au moins un précurseur d'au moins un oxyde d'au moins un métal choisi dans le groupe formé par les métaux du groupes VIII et les métaux du groupe VIB, le(s) précurseur(s) d'au moins un oxyde d'au moins un métal du groupe VIII étant de préférence introduit(s) après ceux du groupe VIB ou en même temps que ces derniers, si le catalyseur contient au moins un métal du groupe VIB et au moins un métal du groupe VIII.

Dans le cas où le catalyseur contient avantageusement au moins un élément du groupe VIB par exemple le molybdène, il est par exemple possible d'imprégner le catalyseur avec une solution contenant au moins un élément du groupe VIB, de sécher, de calciner. L'imprégnation du molybdène peut avantageusement être facilitée par ajout d'acide phosphorique dans les solutions de paramolybdate d'ammonium, ce qui permet d'introduire aussi le phosphore de façon à promouvoir l'activité catalytique.

Les éléments suivants : bore et/ou silicium et/ou phosphore peuvent être introduits dans le catalyseur à tout niveau de la préparation et selon toute technique connue de l'homme du métier.

Une méthode préférée selon l'invention consiste à déposer le ou les éléments promoteurs choisis, par exemple le couple bore-silicium, sur le support mis en forme ou non, de préférence calciné. Pour cela on prépare une solution aqueuse d'au moins un sel de bore tel que le biborate d'ammonium ou le pentaborate d'ammonium en milieu alcalin et en présence d'eau oxygénée et on procède à une imprégnation dite à sec, dans laquelle on remplit le volume des pores du support par la solution contenant par exemple le bore. Dans le cas où l'on dépose par exemple également du silicium, on utilise par exemple une solution d'un composé du silicium de type silicone ou émulsion d'huile silicone.

Le ou les élément(s) promoteur(s) choisi(s) dans le groupe formé par le silicium, le bore et le phosphore peuvent avantageusement être introduits par une ou plusieurs opérations d'imprégnation avec excès de solution sur le précurseur calciné.

La source de bore peut avantageusement être l'acide borique, de préférence l'acide orthoborique H₃BO₃, le biborate ou le pentaborate d'ammonium, l'oxyde de bore, les esters boriques. Le bore peut par exemple être introduit sous la forme d'un mélange d'acide borique, d'eau oxygénée et un composé organique basique contenant de l'azote tels que l'ammoniaque, les amines primaires et secondaires, les amines cycliques, les composés de la famille de la pyridine et des quinoléines et les composés de la famille du pyrrole. Le bore peut être introduit par exemple par une solution d'acide borique dans un mélange eau/alcool.

La source de phosphore préférée est l'acide orthophosphorique H₃PO₄, mais ses sels et esters comme les phosphates d'ammonium conviennent également. Le phosphore peut par exemple être introduit sous la forme d'un mélange d'acide phosphorique et un composé organique basique contenant de l'azote tels que l'ammoniaque, les amines primaires et secondaires, les amines cycliques, les composés de la famille de la pyridine et des quinoléines et les composés de la famille du pyrrole.

De nombreuses sources de silicium peuvent avantageusement être employées. Ainsi, on peut utiliser l'orthosilicate d'éthyle Si(OEt)₄, les siloxanes, les polysiloxanes, les silicones, les émulsions de silicones, les silicates d'halogénures comme le fluorosilicate d'ammonium (NH₄)₂SiF₆ ou le fluorosilicate de sodium Na₂SiF₆. L'acide silicomolybdique et ses sels, l'acide silicotungstique et ses sels peuvent également être avantageusement employés. Le silicium peut avantageusement être ajouté par exemple par imprégnation de silicate d'éthyle en solution dans un mélange eau/alcool. Le silicium peut être ajouté par exemple par imprégnation d'un composé du silicium de type silicone ou l'acide silicique mis en suspension dans l'eau.

Les métaux nobles du groupe VIII du catalyseur de la présente invention peuvent avantageusement être présents en totalité ou partiellement sous forme métallique et/ou oxyde.

Les sources d'éléments nobles du groupe VIII qui peuvent avantageusement être utilisées sont bien connues de l'homme du métier. Pour les métaux nobles on utilise les halogénures, par exemple les chlorures, les nitrates, les acides tels que l'acide chloroplatinique, les hydroxydes, les oxychlorures tels que l'oxychlorure ammoniacal de ruthénium. On peut également avantageusement utiliser les complexes cationiques tels que les sels d'ammonium.

Les catalyseurs ainsi obtenus sont mis en forme sous la forme de grains de différentes formes et dimensions. Ils sont utilisés en général sous la forme d'extrudés cylindriques ou polylobés tels que bilobés, trilobés, polylobés de forme droite ou torsadée, mais peuvent éventuellement être fabriqués et employés sous la forme de poudres concassées, de tablettes, d'anneaux, de billes, de roues. De préférence, les catalyseurs mis en oeuvre dans le procédé selon l'invention ont la forme de sphères ou d'extrudés. Il est toutefois avantageux que le catalyseur se présente sous forme d'extrudés d'un diamètre compris entre 0,5 et 5 mm et plus particulièrement entre 0,7 et 2,5 mm. Les formes sont cylindriques (qui peuvent être creuses ou non), cylindriques torsadés, multilobées (2, 3, 4 ou 5 lobes par exemple), anneaux. La forme cylindrique est avantageusement utilisée de manière préférée, mais toute autre forme peut avantageusement être utilisée.

Le catalyseur mis en forme selon l'invention présente avantageusement généralement une résistance à l'écrasement d'au moins 70 N/cm et de manière préférée supérieure ou égale à 100 N/cm.

Dans le cas où le catalyseur utilisé dans le procédé selon l'invention comprend au moins un métal noble, le métal noble contenu dans ledit catalyseur doit être réduit. La réduction du métal est avantageusement réalisée par le traitement sous hydrogène à une température comprise entre 150°C et 650°C et une pression totale comprise entre 0,1 et 25 MPa. Par exemple, une réduction consiste en un palier à 150°C de deux heures puis une montée en température jusqu'à 450°C à la vitesse de 1°C/min puis un palier de deux heures à 450°C; durant toute cette étape de réduction, le débit d'hydrogène est de 1000 normaux m³ hydrogène par m³ catalyseur et la pression totale maintenue constante à 0,1 MPa. Toute méthode de réduction ex-situ peut avantageusement être envisagée. Dans le cas où le catalyseur utilisé dans le procédé selon l'invention comprend au moins un métal du groupe VIB en combinaison avec au moins un métal non noble du groupe VIII, les métaux sont de préférence utilisés sous leur forme sulfurée. La sulfuration du catalyseur peut etre effectuée in situ ou ex situ par toute méthode connue de l'homme du métier.

### Procédé de conversion

La charge paraffinique constituée par des hydrocarbures ayant un nombre d'atomes de carbone compris entre 9 et 25 et produite à partir de ressources renouvelables, est mise en contact, en présence d'hydrogène avec ledit catalyseur, à des températures et des pressions opératoires permettant avantageusement de réaliser une conversion et de préférence une hydroisomérisation permettant d'atteindre les propriétés à froid visées.

Conformément à l'invention, ledit procédé est effectué à une température comprise entre 150 et 500°C, à une pression comprise entre 0,1 MPa et 15 MPa, à une vitesse spatiale horaire comprise entre 0,1 et 10 h⁻¹ et en présence d'une quantité totale d'hydrogène mélangée à la charge telle que le ratio hydrogène/charge soit compris entre 70 et 2000 Nm³/m³ de charge.

De préférence, ledit procédé est effectué à une température comprise entre 150°C et 450°C, et de manière très préférée, entre 200 et 450°C, à une pression comprise entre 0,2 et 15 MPa, de préférence entre 0,5 et 10 MPa et de manière très préférée, entre 1 et 9 MPa, à une vitesse volumique horaire avantageusement comprise entre 0,2 et 7 h⁻¹ et de manière préférée, entre 0,5 et 5 h⁻¹, et en présence d'une quantité totale d'hydrogène mélangée à la charge telle que le ratio hydrogène/charge soit compris entre 100 et 1500 normaux m³ d'hydrogène par m³ de charge et de préférence entre 150 et 1500 normaux m³ d'hydrogène par m³ de charge.

De préférence, l'effluent issu du procédé de conversion selon l'invention est soumis au moins en partie, et de préférence en totalité, à une ou plusieurs séparations de manière à récupérer une coupe bouillant à une température comprise entre 150 et 370°C. Le but de cette étape est de séparer les gaz du liquide, et notamment, de récupérer les gaz riches en hydrogène pouvant contenir également des légers tels que la coupe C₁ - C₄ et au moins une coupe bouillant à une température comprise entre 150 et 370°C correspondant à une base gazole et/ou une base kérosène et de préférence une base kérosène.

### Exemples

### Exemple 1 : Préparation d'un catalyseur d'hvdroisomérisation C1 (non conforme) Pt-SiAl.

Le catalyseur C1 est un catalyseur contenant un métal noble, le platine, et au moins une silice alumine. Il s'agit d'une silice alumine commerciale sous forme d'extrudés, fournie par la société AXENS. Cette silice alumine contient 35% en poids de silice et 35% poids d'alumine selon les résultats obtenus par Fluorescence X. Ladite silice alumine présente les caractéristiques suivantes :
- une surface spécifique BET comprise de 225 m²/g,
- un volume poreux total, mesuré par porosimétrie au mercure, de 0,4 ml/g,
- un volume poreux, mesuré par porosimétrie au mercure, compris dans les pores de diamètre supérieur à 140 Å de 0,02 ml/g,
- un volume poreux, mesuré par porosimétrie au mercure, compris dans les pores de diamètre supérieurs à 500 Å de 0,01 ml/g,
- diamètre moyen poreux, mesuré par porosimétrie au mercure, de 72 Å,
- une teneur en sodium mesurée par absorption atomique inférieure à 0,025% poids.
Les extrudés de silice alumine sont tout d'abord broyés puis tamisés afin de récupérer une poudre de granulométrie comprise entre 355 et 500 microns. Le dépôt de platine sur la poudre s'effectue ensuite par imprégnation à sec par une solution aqueuse du complexe de Keller Pt(NH₃)₄Cl₂. Après séchage en étuve une nuit à 110°C, la poudre est imprégnée à sec par la solution aqueuse de Pt(NH₃)₄Cl₂, laissée à maturer en maturateur durant typiquement 24 heures à température ambiante puis calcinée en lit traversé sous débit d'air sec fixé à 2 normaux litres par heure et par gramme de solide, à des paliers successifs de température de 150°C durant une heure, 250°C durant une heure, 350°C durant une heure et enfin 520°C durant deux heures. La teneur pondérale en platine mesurée par FX sur le catalyseur fini après calcination est de 0,1% en poids.

### Exemple 2 : Préparation du catalyseur d'hvdroisomérisation C2 (non conforme) Pt-Nu-10.

Le catalyseur C2 est un catalyseur contenant un métal noble, le platine, et une zéolithe Nu-10. La zéolithe Nu-10 est synthétisée selon le protocole décrit dans l'exemple 1 du brevet EP0077624B1 en partant d'un mélange réactionnel présentant la composition molaire suivante:
60 SiO₂; 0,8 Al₂O₃; 8,7 K₂O; 18 DAH; 2470 H₂O
dans laquelle DAH correspond au 1,6 diamino hexane.

La zéolithe brute de synthèse est alors soumise à une calcination en couche mince en four à moufle à 200°C durant deux heures (rampe de montée en température de 2°C/min) puis à 550°C durant douze heures (rampe de montée en température de 1°C/min). Pour obtenir la zéolithe sous sa forme ammonium, la zéolithe calcinée est ensuite échangée avec une solution aqueuse de nitrate d'ammonium 10 M (10 ml de solution par gramme de solide) sous agitation et sous reflux pendant 4 heures. Le solide est ensuite rincé à l'eau distillée et récupéré par centrifugation et séché en couche mince en étuve durant une nuit. L'opération d'échange, rinçage et séchage est effectuée trois fois. Pour obtenir la zéolithe sous sa forme acide (H⁺), la poudre est ensuite calcinée en lit traversé sous débit d'air sec fixé à 2 normaux litres par heure et par gramme de solide à des paliers successifs de température de 150°C durant une heure, 250°C durant une heure, 350°C durant une heure et enfin 550°C durant quatre heures. La zéolithe obtenue présente un rapport atomique Si/Al (déterminé par Fluorescence X) de 31 et une teneur en potassium mesurée par absorption atomique de 0,042% en poids.

Le dépôt de platine sur la poudre s'effectue ensuite par imprégnation à sec par une solution aqueuse du complexe de Keller Pt(NH₃)₄Cl₂. Après séchage en étuve une nuit à 110°C, la zéolithe est imprégnée à sec par la solution aqueuse de Pt(NH₃)₄Cl₂, laissée à maturer en maturateur durant typiquement 24 heures à température ambiante puis calcinée en lit traversé sous débit d'air sec (fixé à 2 normaux litres par heure et par gramme de solide) à des paliers successifs de température de 150°C (durant une heure), 250°C (durant une heure), 350°C (durant une heure) et enfin 520°C (durant deux heures). La teneur pondérale en platine mesurée par FX sur le catalyseur après calcination est de 0,4% en poids.

Finalement le catalyseur C2 est mis en forme par pastillage de la poudre sur presse hydraulique puis broyage et tamisage des pastilles obtenues afin de récupérer une poudre de granulométrie comprise entre 355 et 500 microns.

### Exemple 3 : Préparation du catalyseur d'hvdroisomérisation C3 (conforme) Pt-Nu-10/SiAl.

Le catalyseur C3 est un catalyseur contenant un métal noble, le platine, la zéolithe Nu-10 et la silice alumine. Ce catalyseur est préparé par mélange en broyeur à boules du catalyseur C1 et du catalyseur C2. Après mélange en broyeur à boules le mélange obtenu est mis en forme par pastillage sur presse hydraulique puis broyage et tamisage des pastilles obtenues afin de récupérer une poudre de granulométrie comprise entre 355 et 500 microns. Les quantités de catalyseur C1 et de catalyseur C2 ont été ajustées de manière à obtenir un catalyseur C3 de composition globale : 0,12% poids Pt / 5,97% poids Nu-10 / 93,90% poids silice alumine.

### Exemple 4 : Hvdroisomérisation du n-hexadécane.

Une charge paraffinique synthétique composée de 80% en poids de n-heptane (Carlo Erba, 99% poids) et de 20% en poids de n-hexadécane (Haltermann, 99% poids) est hydroisomérisée sur les différents catalyseurs d'hydroisomérisation en lit traversé dans une unité de test à haut débit, selon le protocole décrit dans la littérature (F.Marques Mota et coll., Prep. Pap. Am. Chem. Soc., Div. Pet. Chem, 2012, 57(1), 145). Il a été vérifié que le solvant n-heptane n'est pas converti dans les conditions opératoires du test avec les catalyseurs C1, C2 et C3. L'effluent hydrocarboné hydroisomérisé est analysé par un système de chromatographie en ligne installé sur l'unité. Les performances catalytiques des catalyseurs sont évaluées à partir des résultats de chromatographie. Avant test catalytique, chaque catalyseur subit une étape de réduction sous débit d'hydrogène dans les conditions opératoires suivantes:
- pression totale : 0,1 MPa,
- débit d'hydrogène: 2000 normaux litres par heure et par litre de catalyseur,
- montée de température ambiante à 450°C à 5°C/minute,
- palier d'une heure à 450°C.

Après l'étape de réduction, la pression et la température sont ajustées aux valeurs souhaitées et la charge est injectée. Les conditions opératoires pour la réaction d'hydroisomérisation du n-hexadécane sont les suivantes:
- pression totale: 0,5 MPa,
- VVH (volume de charge / volume de catalyseur / heure): 11 h⁻¹,
- rapport hydrogène / charge: 1800 normaux litres / litre,
- température: variable.

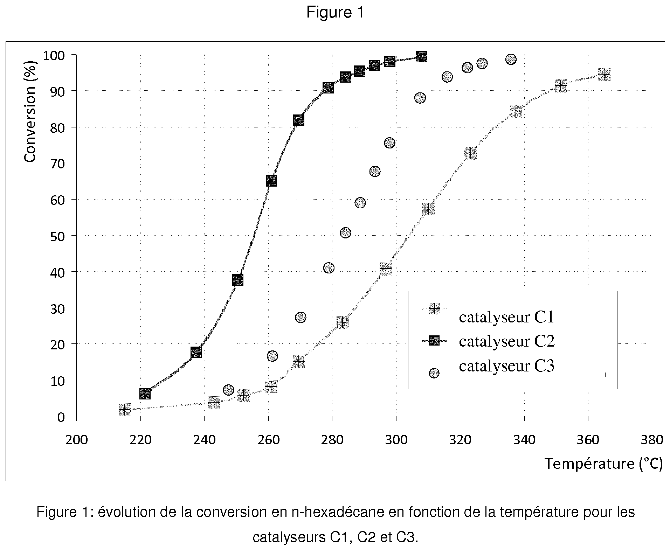

Pour chaque catalyseur, différentes températures de test sont mises en oeuvre afin de faire varier le niveau de conversion du n-hexadécane. Pour le catalyseur C1, la température est ainsi variée entre 210 et 370°C, pour le catalyseur C2 la température est variée entre 220 et 310°C et pour le catalyseur C3 la température est variée entre 240 et 340°C. La figure 1 reporte l'évolution de la température en fonction de la conversion pour les trois catalyseurs. Comme attendu le catalyseur C3 présente une activité intermédiaire entre celle du catalyseur C1 et celle du catalyseur C2.

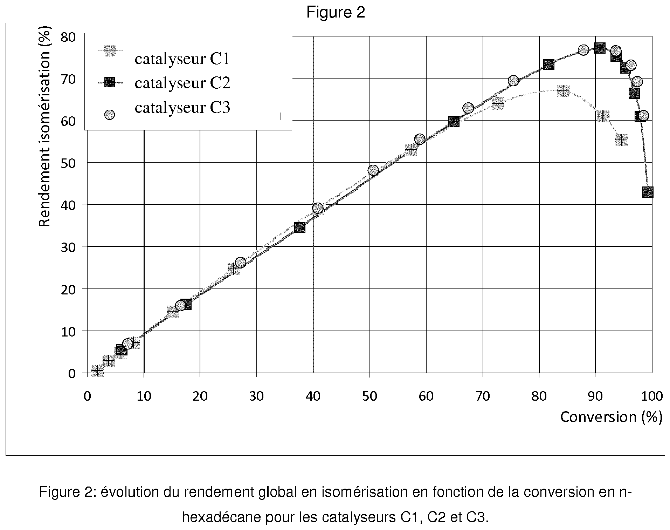

La figure 2 reporte l'évolution du rendement global en isomérisation (isomères monobranchés et multibranchés du n-hexadécane) en fonction de la conversion du n-hexadécane pour les trois catalyseurs. Comme attendu, le catalyseur C2 à base de zéolithe Nu-10 permet d'obtenir des rendements en isomérisation plus importants que le catalyseur C1 à base de silice alumine pour des conversions supérieures à 70%. De manière surprenante le catalyseur C3 à base de zéolithe Nu-10 et de silice alumine ne présente pas un comportement intermédiaire entre les catalyseurs C1 et C2 mais permet l'obtention de rendements en isomérisation comparables à ceux observés avec le catalyseur C2.

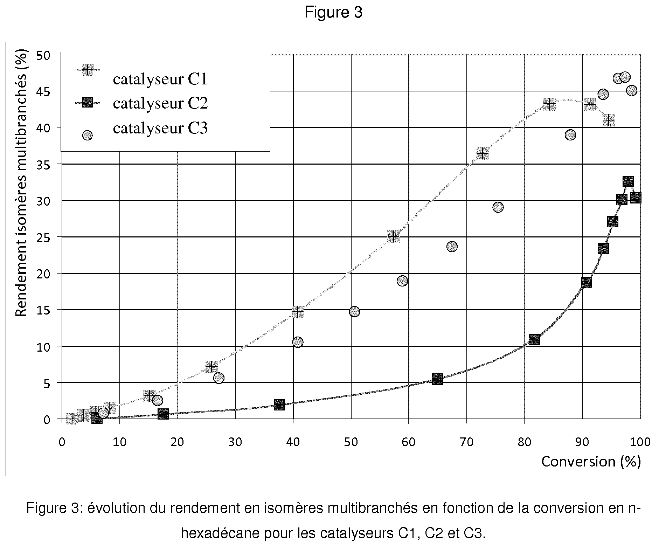

La figure 3 reporte l'évolution du rendement en isomères multibranchés en fonction de la conversion du n-hexadécane pour les trois catalyseurs. Sur la gamme de conversion étudiée, le catalyseur C1 à base de silice alumine permet d'obtenir des rendements en isomères multibranchés supérieurs au catalyseur C2 à base de zéolithe Nu-10. De manière surprenante le catalyseur C3 ne présente pas un comportement intermédiaire entre C1 et C2 mais permet d'obtenir les rendements en isomères multibranchés les plus élevés pour des conversions supérieures à 90%.

Ainsi, le catalyseur C3 permet d'une part d'obtenir des rendements en isomérisation comparables à ceux obtenus sur le catalyseur C2 et supérieurs à ceux obtenus avec le catalyseur C1 pour des conversions supérieures à 70% et d'autre part d'obtenir les rendements en isomères multibranchés les plus élevés pour des conversions supérieures à 90%. Le tableau 1 reporte ainsi les performances des catalyseurs C1, C2 et C3 pour les rendements maxima en isomérisation globale (Rendementₘₐₓ *iso*-C₁₆ dans le tableau 1) obtenus sur chacun des catalyseurs.

Comparativement à C1, C3 permet d'obtenir un rendement global en isomérisation maximal plus élevé de 10 points.

Comparativement à C2, pour un même rendement global en isomérisation maximal de 77%, C3 permet l'obtention d'un rendement en isomères multibranchés plus élevé de 20 points (Rendement *iso*-C₁₆ multibranchés dans le tableau 1) .

Par ailleurs, l'utilisation du catalyseur selon l'invention C3 permet l'obtention d'un rendement en craquage plus faible (Rendement en craquage dans le tableau 1) que les catalyseurs C1 et C2 ce qui permet de limiter la production de produits craqués légers.

**Tableau 1: performances des catalyseurs C1, C2 et C3 pour les rendements maxima en isomérisation global obtenus sur chacun des catalyseurs.**

| Catalyseur | **C1** | **C2** | **C3** |
|---|---|---|---|
| Rendement ₘₐₓ *iso-*C₁₆ (%) | 67 | 77 | 77 |
| Rendement *iso-*C₁₆ monobranchés (%) | 24 | 58 | 38 |
| Rendement *iso-*C₁₆ multibranchés (%) | 43 | 19 | 39 |
| Rendement en craquage (%) | 17 | 14 | 11 |

## Revendications

1. Procédé de conversion d'une charge paraffinique constituée par des hydrocarbures ayant un nombre d'atomes de carbone compris entre 9 et 25, ladite charge paraffinique étant produite à partir de ressources renouvelables, à l'exclusion des charges paraffiniques obtenues par un procédé mettant en jeu une étape de valorisation par la voie Fischer-Tropsch, ledit procédé mettant en oeuvre un catalyseur comprenant au moins un métal hydro-déshydrogénant choisi dans le groupe formé par les métaux du groupe VIB et du groupe VIII de la classification périodique, pris seuls ou en mélange et un support comprenant au moins une zéolithe Nu-10 et au moins une silice alumine, la silice alumine utilisée en tant que support dudit catalyseur contenant une quantité supérieure à 5% poids et inférieure ou égale à 95% poids de silice, ledit procédé opérant à une température comprise entre 150 et 500°C, à une pression comprise entre 0,1 MPa et 15 MPa, à une vitesse spatiale horaire comprise entre 0,1 et 10 h⁻¹ et en présence d'une quantité totale d'hydrogène mélangée à la charge telle que le ratio hydrogène/charge soit compris entre 70 et 2000 Nm³/m³ de charge,
dans lequel ledit catalyseur contient un liant, ledit catalyseur comprenant une teneur totale en zéolithe Nu-10 et en silice alumine comprise entre 1,5% et 94,5% en poids par rapport à la masse totale dudit catalyseur, la teneur en poids de la zéolithe Nu-10 étant inférieure à la teneur en poids de la silice alumine ; ou
dans lequel ledit catalyseur ne contient pas de liant, ledit catalyseur comprenant une teneur totale en zéolithe Nu-10 et en silice alumine d'au moins 50% en poids par rapport à la masse totale dudit catalyseur, la teneur en poids de la zéolithe Nu-10 étant inférieure à la teneur en poids de la silice alumine.

2. Procédé selon la revendication 1 dans lequel ladite charge paraffinique est constituée par des hydrocarbures ayant un nombre d'atomes de carbone compris entre 10 et 22.

3. Procédé selon l'une des revendications 1 ou 2 dans lequel ladite charge paraffinique est produite à partir de ressources renouvelables choisies parmi les huiles végétales, les huiles d'algues ou algales, les huiles de poissons et les graisses d'origine végétale ou animale, ou des mélanges de telles charges.

4. Procédé selon l'une des revendications 1 à 3 dans lequel ledit procédé est un procédé d'hydroisomérisation.

5. Procédé selon l'une des revendications 1 à 4 dans lequel les éléments du groupe VIII sont choisis parmi le cobalt, le nickel, le platine et le palladium, pris seul ou en mélange.

6. Procédé selon la revendication 5 dans lequel la teneur en métal noble dudit catalyseur est comprise entre 0,01 et 5% en poids par rapport à la masse totale dudit catalyseur.

7. Procédé selon l'une des revendications 1 à 6 dans lequel les éléments du groupe VIB sont choisis parmi le tungstène et le molybdène, pris seuls ou en mélange.

8. Procédé selon l'une des revendications 1 à 5 et 7 dans lequel la teneur en métal du groupe VIB est comprise entre 5 et 40% en poids d'oxyde par rapport à la masse totale dudit catalyseur, et la teneur en métal non noble du groupe VIII est comprise entre 0,5 et 10% en poids d'oxyde par rapport à la masse totale dudit catalyseur.

9. Procédé selon l'une des revendications 1 à 8 dans lequel la silice alumine utilisée dans le support dudit catalyseur contient une quantité supérieure à 5% poids et inférieure ou égale à 95% poids de silice, et présente les caractéristiques texturales suivantes :
- un diamètre moyen poreux, mesuré par porosimétrie au mercure, compris entre 20 et 140 Å,
- un volume poreux total, mesuré par porosimétrie au mercure, compris entre 0,1 ml/g et 0,5 ml/g,
- un volume poreux total, mesuré par porosimétrie azote, compris entre 0,1 ml/g et 0,5 ml/g,
- une surface spécifique BET comprise entre 100 et 550 m²/g,
- un volume poreux, mesuré par porosimétrie au mercure, compris dans les pores de diamètre supérieur à 140 Å inférieur à 0,1 ml/g ,
- un volume poreux, mesuré par porosimétrie au mercure, compris dans les pores de diamètre supérieurs à 500 Å inférieur à 0,1 ml/g,
- un diagramme de diffraction X qui contient au moins les raies principales caractéristiques d'au moins une des alumines de transition comprise dans le groupe composé par les alumines alpha, rhô, chi, eta, gamma, kappa, thêta et delta.

10. Procédé selon l'une des revendications 1 à 8 dans lequel ledit catalyseur contient un liant, ledit liant étant choisi dans le groupe formé par l'alumine, la silice, les argiles, l'oxyde de titane, l'oxyde de bore et la zircone, pris seuls ou en mélange.

11. Procédé selon la revendication 10 dans lequel ledit catalyseur comprend de 5 à 98% en poids de liant par rapport à la masse totale dudit catalyseur.

12. Procédé selon l'une des revendications 1 à 11 dans lequel ledit procédé est effectué à une température comprise entre 150°C et 450°C, à une pression comprise entre 0,2 et 15 MPa, à une vitesse volumique horaire comprise entre 0,2 et 7 h⁻¹ et en présence d'une quantité totale d'hydrogène mélangée à la charge telle que le ratio hydrogène/charge soit compris entre 100 et 1500 normaux m³ d'hydrogène par m³ de charge.

## Patentansprüche

1. Verfahren zur Umwandlung einer Paraffincharge, die aus Kohlenwasserstoffen mit einer Anzahl von Kohlenstoffatomen zwischen 9 und 25 besteht, wobei die Paraffincharge aus erneuerbaren Ressourcen hergestellt wird, unter Ausschluss von Paraffinchargen, die durch ein Verfahren erhalten werden, das einen Schritt der Fischer-Tropsch-Valorisierung verwendet, wobei das Verfahren einen Katalysator verwendet, umfassend mindestens ein Hydro-Dehydrierungsmetall, ausgewählt aus der Gruppe, die gebildet wird aus Metallen der Gruppe VIB und der Gruppe VIII des Periodensystems, allein oder in Mischung, und einen Träger, umfassend mindestens einen Zeolith Nu-10 und mindestens ein Siliciumdioxid-Aluminiumoxid, wobei das Siliciumdioxid-Aluminiumoxid, das als Träger des Katalysators verwendet wird, eine Menge von größer als 5 Gew.-% und kleiner oder gleich 95 Gew.-% Siliciumdioxid enthält, wobei das Verfahren betrieben wird bei einer Temperatur zwischen 150 und 500°C, bei einem Druck zwischen 0,1 MPa und 15 MPa, bei einer Raumgeschwindigkeit pro Stunde zwischen 0,1 und 10 h⁻¹ und in Anwesenheit einer Gesamtmenge an Wasserstoff, gemischt mit der Charge, derart, dass das Verhältnis von Wasserstoff/Charge zwischen 70 und 2000 Nm³/m³ Charge beträgt, wobei der Katalysator ein Bindemittel enthält, wobei der Katalysator einen Gesamtgehalt an Zeolith Nu-10 und an Siliciumdioxid-Aluminiumoxid zwischen 1,5 Gew.-% und 94,5 Gew.-%, bezogen auf die Gesamtmasse des Katalysators, umfasst, wobei der Gewichtsanteil an Zeolith Nu-10 kleiner ist als der Gewichtsanteil an Siliciumdioxid-Aluminiumoxid; oder
wobei der Katalysator kein Bindemittel enthält, wobei der Katalysator einen Gesamtgehalt an Zeolith Nu-10 und an Siliciumdioxid-Aluminiumoxid von weniger als 50 Gew.-%, bezogen auf die Gesamtmasse des Katalysators, umfasst, wobei der Gewichtsanteil an Zeolith Nu-10 kleiner ist als der Gewichtsanteil an Siliciumdioxid-Aluminiumoxid.

2. Verfahren nach Anspruch 1, wobei die Paraffincharge aus Kohlenwasserstoffen mit einer Anzahl von Kohlenstoffatomen zwischen 10 und 22 besteht.

3. Verfahren nach einem der Ansprüche 1 oder 2, wobei die Paraffincharge aus erneuerbaren Ressourcen erzeugt wird, ausgewählt aus Pflanzenölen, Algen- oder Algenblütenölen, Fischölen und Fetten pflanzlichen oder tierischen Ursprungs, oder Mischungen solcher Chargen.

4. Verfahren nach einem der Ansprüche 1 bis 3, wobei das Verfahren ein Hydroisomerisierungsverfahren ist.

5. Verfahren nach einem der Ansprüche 1 bis 4, wobei die Elemente der Gruppe VIII ausgewählt werden aus Kobalt, Nickel, Platin und Palladium, allein oder in Mischung.

6. Verfahren nach Anspruch 5, wobei der Gehalt an Edelmetall des Katalysators zwischen 0,01 und 5 Gew.-%, bezogen auf die Gesamtmasse des Katalysators, liegt.

7. Verfahren nach einem der Ansprüche 1 bis 6, wobei die Elemente der Gruppe VIB ausgewählt werden aus Wolfram und Molybdän, allein oder in Mischung.

8. Verfahren nach einem der Ansprüche 1 bis 5 und 7, wobei der Gehalt an Metall der Gruppe VIB zwischen 5 und 40 Gew.-% Oxid, bezogen auf die Gesamtmasse des Katalysators, liegt, und der Gehalt an NichtEdelmetall der Gruppe VIII zwischen 0,5 und 10 Gew.-% Oxid, bezogen auf die Gesamtmasse des Katalysators, liegt.

9. Verfahren nach einem der Ansprüche 1 bis 8, wobei das Siliciumdioxid-Aluminiumoxid, das in dem Träger des Katalysators verwendet wird, eine Menge von größer als 5 Gew.-% und kleiner oder gleich 95 Gew.-% Siliciumdioxid enthält, und die folgenden Texturcharakteristiken aufweist:
- einen porösen mittleren Durchmesser, gemessen durch Quecksilber-Porosimetrie, zwischen 20 und 140 Å,
- ein poröses Gesamtvolumen, gemessen durch Quecksilber-Porosimetrie, zwischen 0,1 ml/g und 0,5 ml/g,
- ein poröses Gesamtvolumen, gemessen durch Stickstoff-Porosimetrie, zwischen 0,1 ml/g und 0,5 ml/g,
- eine spezifische Oberfläche BET zwischen 100 und 550 m²/g,
- ein poröses Volumen, gemessen durch Quecksilber-Porosimetrie, in den Poren mit einem Durchmesser von größer als 140 Å, kleiner als 0,1 ml/g,
- ein poröses Volumen, gemessen durch Quecksilber-Porosimetrie, in den Poren mit einem Durchmesser von größer als 500 Å, kleiner als 0,1 ml/g,
- ein X-Beugungsdiagramm, das mindestens die charakteristischen Hauptlinien mindestens eines der Übergangs-Aluminiumoxide enthält, in der Gruppe bestehend aus den Aluminiumoxiden alpha, rho, eta, gamma, kappa, theta und delta.

10. Verfahren nach einem der Ansprüche 1 bis 8, wobei der Katalysator ein Bindemittel enthält, wobei das Bindemittel ausgewählt wird aus der Gruppe, die gebildet wird aus Aluminiumoxid, Siliciumdioxid, Tonen, Titanoxid, Boroxid und Zirkon, allein oder in Mischung.

11. Verfahren nach Anspruch 10, wobei der Katalysator 5 bis 98 Gew.-% Bindemittel, bezogen auf die Gesamtmasse des Katalysators, umfasst.

12. Verfahren nach einem der Ansprüche 1 bis 11, wobei das Verfahren durchgeführt wird bei einer Temperatur zwischen 150°C und 450°C, bei einem Druck zwischen 0,2 und 15 MPa, bei einer Volumengeschwindigkeit pro Stunde zwischen 0,2 und 7 h⁻¹ und in Anwesenheit einer Gesamtmenge an Wasserstoff, gemischt mit der Charge, derart, dass das Verhältnis von Wasserstoff/Charge zwischen 100 und 1500 normalen m³ Wasserstoff pro m³ Charge liegt.

## Claims

1. A process for the conversion of a paraffinic feed comprising hydrocarbons containing in the range 9 to 25 carbon atoms, wherein said paraffinic feed is produced from renewable resources and excludes paraffinic feeds obtained by a process comprising a step for upgrading by a Fischer-Tropsch method, said process employing a catalyst comprising at least one hydro-dehydrogenating metal selected from the group formed by metals from group VIB and from group VIII of the periodic classification of the elements, used alone or as a mixture, and a support comprising at least one Nu-10 zeolite and at least one silica-alumina, the silica-alumina used as a support for said catalyst containing an amount of silica of more than 5% by weight and less than or equal to 95% by weight, said process operating at a temperature in the range 150°C to 500°C, at a pressure in the range 0.1 MPa to 15 MPa, at an hourly space velocity in the range 0.1 to 10 h⁻¹ and in the presence of a total quantity of hydrogen mixed with the feed such that the hydrogen/feed ratio is in the range 70 to 2000 Nm³/m³ of feed,
wherein said catalyst contains a binder, said catalyst comprising a total amount of Nu-10 zeolite and silica-alumina in the range 1.5% to 94.5% by weight with respect to the total mass of said catalyst, the content by weight of Nu-10 zeolite being less than the content by weight of silica-alumina; or
wherein said catalyst does not contain any binder, said catalyst comprising a total amount of Nu-10 zeolite and silica-alumina of at least 50% by weight with respect to the total mass of said catalyst, the content by weight of Nu-10 zeolite being less than the content by weight of silica-alumina.

2. The process as claimed in one of claims 1 or 2, wherein said paraffinic feed is constituted by hydrocarbons containing in the range 10 to 22 carbon atoms.

3. The process as claimed in claim 1, wherein said paraffinic feed is produced from renewable resources selected from vegetable oils, oils from algae or algals, fish oils and fats of animal or vegetable origin, or mixtures of such feeds.

4. The process as claimed in one of claims 1 to 3, wherein said process is a hydroisomerization process.

5. The process as claimed in one of claims 1 to 4, wherein the elements from group VIII are selected from cobalt, nickel, platinum and palladium, used alone or as a mixture.

6. The process as claimed in claim 5, wherein the quantity of noble metal of said catalyst is in the range 0.01% to 5% by weight with respect to the total mass of said catalyst.

7. The process as claimed in one of claims 1 to 6, wherein the elements from group VIB are selected from tungsten and molybdenum, used alone or as a mixture.

8. The process as claimed in one of claims 1 to 5 and 7, wherein the quantity of metal from group VIB is in the range 5% to 40% by weight of oxide with respect to the total mass of said catalyst, and the quantity of non-noble metal from group VIII is in the range 0.5% to 10% by weight of oxide with respect to the total mass of said catalyst.

9. The process as claimed in one of claims 1 to 8, wherein the silica-alumina used in the support for said catalyst contains a quantity of more than 5% by weight and less than or equal to 95% by weight of silica and has the following textural characteristics:
• a mean pore diameter, measured by mercury porosimetry, in the range 20 to 140 Å;
• a total pore volume, measured by mercury porosimetry, in the range 0.1 mL/g to 0.5 mL/g;
• a total pore volume, measured by nitrogen porosimetry, in the range 0.1 mL/g to 0.5 mL/g;
• a BET specific surface area in the range 100 to 550 m²/g;
• a pore volume, measured by mercury porosimetry, included in pores with a diameter of more than 140 Å, of less than 0.1 mL/g;
• a pore volume, measured by mercury porosimetry, included in pores with a diameter of more than 500 Å, of less than 0.1 mL/g;
• an X ray diffraction pattern which contains at least the principal characteristic peaks of at least one of the transition aluminas included in the group composed of alpha, rho, chi, eta, gamma, kappa, theta and delta aluminas.

10. The process as claimed in one of claims 1 to 8, wherein said catalyst contains a binder, said binder being selected from the group formed by alumina, silica, clays, titanium oxide, boron oxide and zirconia, used alone or as a mixture.

11. The process as claimed in claim 10, wherein said catalyst comprises 5% to 98% by weight of binder with respect to the total mass of said catalyst.

12. The process as claimed in one of claims 1 to 11, wherein said process is carried out at a temperature in the range 150°C to 450°C, at a pressure in the range 0.2 to 15 MPa, at an hourly space velocity in the range 0.2 to 7 h⁻¹ and in the presence of a total quantity of hydrogen mixed with the feed such that the hydrogen/feed ratio is in the range 100 to 1500 normal m³ of hydrogen per m³ of feed.
